(19) Europäisches Patentamt
European Patent Office
Office européen des brevets

(11) EP 4 140 511 A1

(12) **EUROPEAN PATENT APPLICATION**
published in accordance with Art. 153(4) EPC

(43) Date of publication:
**01.03.2023 Bulletin 2023/09**

(21) Application number: **21792008.1**

(22) Date of filing: **23.04.2021**

(51) International Patent Classification (IPC):
***A61L 27/36*** *(2006.01)* ***A61L 27/24*** *(2006.01)*
***A61L 33/00*** *(2006.01)* ***C08F 289/00*** *(2006.01)*
***C08F 220/60*** *(2006.01)* ***C08F 220/36*** *(2006.01)*
***C08F 230/02*** *(2006.01)*

(86) International application number:
**PCT/CN2021/089448**

(87) International publication number:
**WO 2021/213516 (28.10.2021 Gazette 2021/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.04.2020 CN 202010331255**

(71) Applicant: **Venus MedTech (HangZhou), Inc.**
**Hangzhou, Zhejiang 310052 (CN)**

(72) Inventors:
- **WANG, Yunbing**
**Hangzhou, Zhejiang 310052 (CN)**
- **YANG, Fan**
**Hangzhou, Zhejiang 310052 (CN)**
- **GUO, Gaoyang**
**Hangzhou, Zhejiang 310052 (CN)**

(74) Representative: **KIPA AB**
**Drottninggatan 11**
**252 21 Helsingborg (SE)**

# (54) ANTICOAGULATION AND ANTICALCIFICATION BIOLOGICAL MATERIAL, AND PREPARATION METHOD THEREFOR

(57) Provided are an anticoagulation and anticalcification biological material and a preparation method therefor. The preparation method includes the following steps: introducing, on a biological tissue, a polymerizable reactive group, and undergoing free radical copolymerization with a zwitterion. In the present invention, by introducing a reactive group capable of free radical polymerization to a biological tissue and undergoing free radical copolymerization with a zwitterionic monomer, collagen in the biological tissue is crosslinked at multiple sites by means of a polymer, thereby achieving sufficient crosslinking within and between collagen fibers, improving the stability of the biological tissue, and prolonging the service life of the biological tissue. Moreover, a zwitterion is introduced to the surface of the biological tissue, to improve the anticoagulation performance, promote the in-situ endothelialization of a biological valve, and prevent the calcium element deposition.

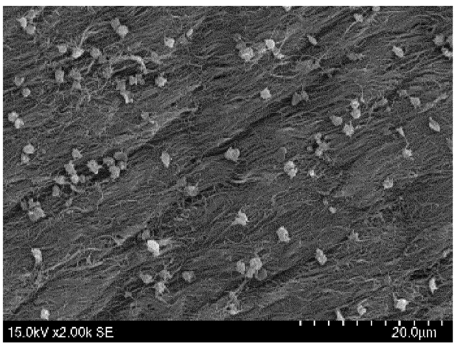


FIG. 3



Processed by Luminess, 75001 PARIS (FR)

## Description

TECHNICAL FIELD

The present invention relates to the technical field of medical materials, and particularly to an anticoagulation and anticalcification biological material and a preparation method therefor.

BACKGROUND

Xenogeneic biological tissues have physicochemical properties similar to those of human tissues, and thus are widely used as natural biological materials for implantation in the human body to replace or repair damaged human tissues or organs, particularly used for repairing soft tissues, for example, as artificial blood vessels, artificial valves, vascular patches, dural patches, hernia patches, anti-adhesion membranes, soft tissue fillers, artificial skin and ventricular assist devices. The xenogeneic biological tissues, if directly implanted into the human body without treatment, will be degraded rapidly due to immune rejection, and lose their mechanical performances. For the sake of long-acting performances, the biological tissues are typically chemically cross-linked to remain stable in the human body for a long time. At present, the xenogeneic biological tissues are generally immobilized by cross-linking with glutaraldehyde. However, the biological tissues cross-linked with glutaraldehyde have obvious inflammatory reactions and cross-linking instability, and suffer from the problems of degradation and calcification upon long-term implantation in the body, causing hardening and embrittling, decreased mechanical performances, loss of normal physiological functions of the implanted tissues. Edwards' technique and Medtronic's technique can reduce the calcification of glutaraldehyde-crosslinked biological tissues, but the problems of poor biocompatibility caused by cross-linking with glutaraldehyde cannot be completely eliminated. When the biological tissues are cross-linked with other compounds such as epoxy compounds, carbodiimides, Genipin and other new cross-linking agents, although the biocompatibility and calcification problems can be alleviated, the thrombogenicity problem cannot be overcome. When these tissues are used as a blood contact material, the large number of collagen molecules present in the biological tissue promote the coagulation. Therefore, neither cross-linking with glutaraldehyde nor cross-linking with non-glutaraldehyde can solve the thrombogenesis problem when the biological tissues are used as a blood contact material.

To solve the problems of thrombogenesis and immune rejection associated with the foreign implants in the prior art, the implant surface is usually covalently modified with a functional molecule, to reduce the interaction with the human body, and avoid the coagulation and the attack by the immune system. The covalent modification is beneficial to the maintenance of functional stability for a long period of time, but readily lead to the inactivation of functional molecules. Moreover, heavily depending on the modification site and modification density, the covalent modification often causes the modified surface to fail to achieve a desired anticoagulation or immune evasion effects. The biological tissues are highly sensitive to the modification conditions, and the traditional modification methods often require harsh conditions and have a low modification efficiency. Therefore, the problems of calcification, coagulation, immune reactions and stability associated with the biological tissue implants cannot be solved simultaneously in the prior art; or complex steps are involved when these problems are solved, so biological tissues with excellent comprehensive performances cannot be obtained.

## SUMMARY

In view of the above problems in the prior art, the present invention provides an anticoagulation and anticalcification biological material and a preparation method therefor. the biological material has excellent comprehensive performances, exhibiting excellent anticoagulation and anti-calcification performances, immune reactions and stability.

To achieve the above object, the following technical solutions are adopted in the present invention.

A method for preparing an anticoagulation and anticalcification biological material includes the following steps: introducing, on a biological tissue, a polymerizable reactive group, and undergoing free radical copolymerization with a zwitterion. The zwitterionic monomer has a structural formula as below:

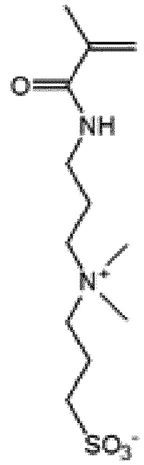, 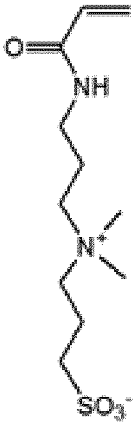, 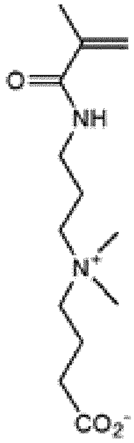, 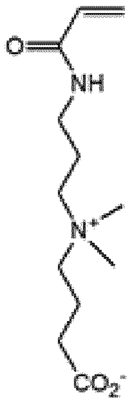, 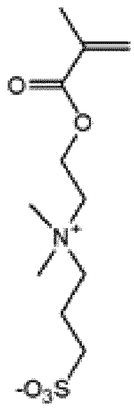, 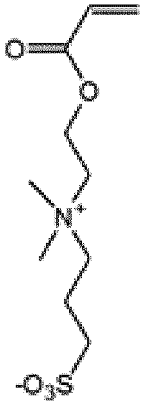, 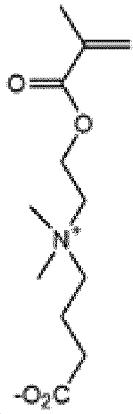,

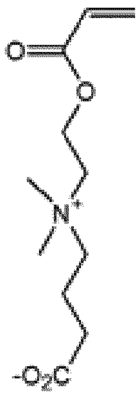 or 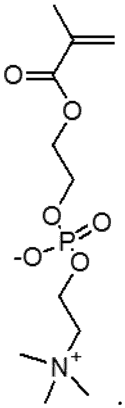.

The compounds represented by the above structural formulas are respectively:

N,N-dimethyl-N-methacrylamidopropyl-methanesulfonate;
N,N-dimethyl-N-acrylamidopropyl-methanesulfonate;
N,N-dimethyl-N-methacrylamidopropyl-methanecarboxylate;
N,N-dimethyl-N-methacrylamidopropyl-methanecarboxylate;
[2-(methacryloyloxy)ethyl]dimethyl-(3-sulfopropyl)ammonium hydroxide (SBMA);
[2-(acryloyloxy)ethyl]dimethyl-(3-sulfopropyl)ammonium hydroxide;
3-[[2-(methacryloyloxy)ethyl]dimethyl ammonium]propionate (CBMA);
3-[[2-(acryloyloxy)ethyl]dimethyl ammonium]propionate; and
2-methacryloyloxyethylphosphorylcholine (MPC).

Preferably, the zwitterion is [2-(methylacryloyloxy)ethyl]dimethyl-(3-sulfopropyl)ammonium hydroxide (SBMA), 3-[[2-(Methylacryloyloxy)ethyl]dimethyl ammonium]propionate (CBMA) or 2-methylacryloyloxyethylphosphorylcholine (MPC).

Further, the biological tissue introduced with the reactive group is added to a zwitterion solution having a final concentration of 20-500 mM, and an initiator is added to initiate the polymerization, to obtain the anticoagulation and anticalcification biological material.

Further, the biological tissue introduced with the reactive group is added to a zwitterion solution and soaked at 35 to 40°C, and then an initiator is added to initiate the polymerization, to obtain the anticoagulation and anticalcification biological material. The time of soaking at 35 to 40°C may be 8 to 24 hrs, for example, 10 to 16 hrs, such as 12 hrs. The specific operation may be soaking overnight.

Further, the biological tissue is soaked in deionized water, and then the reactive group is added, to provide a concentration of the reactive group of 3-10% by volume.

Further, the biological tissue is reacted with the reactive group at room temperature for 12 to 36 hrs, and then the biological tissue is washed.

Further, the biological tissue is soaked in deionized water, and then the reactive group is added to provide a concentration of the reactive group of 4% by volume. and then reacted at room temperature for 24 hrs.

Further, a zwitterion solution is added to the washed biological tissue, to provide a final concentration of the zwitterion of 500 mM, and the biological tissue is soaked at 37°C. The time of soaking at 37°C may be 8 to 24 hrs, for example, 10 to 16 hrs, such as 12 hrs. The specific operation may be soaking overnight.

The preparation process is specifically: soaking the biological tissue in deionized water, then adding the reactive group, to provide a concentration of the reactive group of 3 to 10% by volume, reacting at room temperature for 12 to 36 hrs, washing the biological tissue, adding a zwitterion monomer solution to the washed biological tissue to provide a final concentration of the zwitterion monomer of 20 to 500 mM, soaking overnight at 35 to 40°C, and then adding an initiator to initiate the polymerization to obtain the biological material.

The collagen molecule of biological tissues is composed of amino acids, and contains a large amount of free amino groups. The reactive group contains a group that can react with the amino group and also a double bond. The reactive group reacts with free amino group in the biological tissue, to connect the reactive group to the biological tissue through a chemical bond, and the double bond can participate in the subsequent polymerization reaction.

The zwitterion contains a cationic group and an anionic group, and is generally neutrally charged. The zwitterion contains a hydrophilic group such as a sulfonic acid group and a carboxylic acid group, to improve the hydrophilicity. The zwitterion also contains a double bond.

The double bond in the reactive group reacts with the double bond in the zwitterion by free radical reaction in the presence of the initiator, so the reactive group is directly chemically attached to the zwitterion. Moreover, in the presence of the initiator. zwitterion itself will also undergo polymerization, and the collagen molecule in the biological tissue is cross-linked through the polymerization of the zwitterion. Since the polymer with a longer molecular chain structure is cross-linked, long-distance cross-linking is achieved.

Generally, the covalent attachment of a functional molecule to the biological tissue requires the use of a functional group on the functional molecule. The functional group will affect the activity of the functional molecule to some extent. In the present disclosure, the reactive group is grafted to the biological tissue through a chemical bond, and the reactive group is chemically attached to the zwitterion compound through free radical polymerization of the double bond. Free radical polymerization is characterized in that one double bond can be used to react with the zwitterion compound constantly, to achieve an effect that multiple zwitterion compound molecules are introduced by one double bond, so the reactive group is unlikely to be inactivated. Moreover, the free radical polymerization is highly reactive, and can accomplish the covalent bonding of the functional molecule more efficiently compared with general chemical reactions. Further, the reactive group is then added to provide a concentration of the reactive group of 4% by volume. and reacted at room temperature for 24 hrs.

Further, a zwitterion solution is added to the washed biological tissue, to provide a final concentration of the zwitterion of 500 mM, and the biological tissue is soaked overnight at 37°C.

Further, the biological tissue is pericardium, aortic root, aortic valve, pulmonary artery root, pulmonary artery valve, tendon, ligament, skin, dura mater, peritoneum, blood vessels, pleura, septum, mitral or tricuspid valve.

Further, the reactive group is methacrylic anhydride, acrylamide, methacrylamide, acrylate, methacrylate or allyl.

Further, the zwitterion is attached to the biological tissue through an amide bond at more than two sites of attachment with the biological tissue.

Further, the zwitterion accounts for 10 to 30% by weight based on the total weight of the anticoagulation and anticalcification biological material; and preferably the zwitterion accounts for 24.6% by weight based on the total weight of the anticoagulation and anticalcification biological material.

Further, when the initiator initiates the polymerization, the reaction temperature is not higher than 50°C, and preferably 37°C.

Further, the initiator is a thermal initiator or a photoinitiator. The thermal initiator is an organic peroxide initiator, an inorganic peroxide initiator, an azo initiator, or a redox initiator, and preferably is a redox initiator such as an ammonium persulfate/sodium bisulfite system. The photoinitiator is IrgacureD-2959.

The present application also provides an anticoagulation and anticalcification biological material prepared by any of the above-mentioned preparation methods.

The anticoagulation and anticalcification biological material and the preparation method therefor provided in the present invention have the following beneficial effects.

The untreated biological tissues will be degraded under the action of enzymes after implantation, and thus are unable to maintain their functions for a long time. In the present invention, a reactive group capable of free radical polymerization is introduced to a biological tissue, which undergoes free radical copolymerization with a zwitterionic monomer, so that collagen in the biological tissue is crosslinked at multiple sites by means of a polymer, thereby achieving sufficient crosslinking within and between collagen fibers, improving the stability of the biological tissue, and prolonging the service life of the biological tissue. Moreover, the zwitterion is introduced to the surface of the biological tissue. The zwitterion is a hydrophilic polymer having anionic and cationic groups, and forms a firm hydration layer on the surface

of the biological tissue through strong ionic solvation and hydrogen bonding. The water molecules in the hydration layer are in a highly free state and can hinder the adsorption of proteins, to block the initiation of the downstream coagulation pathway, improve the anticoagulation performance, and promote the in-situ endothelialization of biological valves, thereby preventing the calcium deposition.

## BRIEF DESCRIPTION OF THE DRAWINGS

FIG. 1 is a flow chart illustrating a preparation of an antithrombotic and anticalcification biological tissue.

FIG. 2 is a schematic diagram showing the mechanism of preparation of an antithrombotic and anticalcification functionalized biological tissue.

FIG. 3 is an SEM image showing the platelet adsorption on MPC-20 biological tissue.

FIG. 4 is an SEM image showing the platelet adsorption on MPC-500 biological tissue.

FIG. 5 is an SEM image showing the platelet adsorption on SBMA-20 biological tissue.

FIG. 6 is an SEM image showing the platelet adsorption on SBMA-500 biological tissue.

FIG. 7 is an SEM image showing the platelet adsorption on an unmodified biological tissue.

FIG. 8 is an SEM image showing the platelet adsorption on a glutaraldehyde-crosslinked biological tissue.

FIG. 9 is a fluorescent micrograph showing endothelial cells grown on SBMA-500 biological tissue.

FIG. 10 is a fluorescent micrograph showing endothelial cells grown on an unmodified biological tissue.

FIG. 11 is a fluorescent micrograph showing endothelial cells grown on a glutaraldehyde-crosslinked biological tissue.

## DETAILED DESCRIPTION OF THE EMBODIMENTS

### Example 1

A method for preparing an anticoagulation and anticalcification biological material is provided. The preparation process is shown in FIG. 1, and the mechanism of preparation is shown in FIG. 2, which specifically includes the steps as follows:

washing a fresh porcine pericardium thoroughly with deionized water, and then adding deionized water to completely submerge the tissue; adding methacrylic anhydride dropwise with stirring in an ice bath at 4°C, to obtain a final concentration of the acid anhydride of 4% (v/v), with the solution maintained at pH 7 with a sodium hydroxide solution; and after that, the reaction was continued at room temperature for 24 hrs; thoroughly washing the porcine pericardium modified with methacrylic anhydride, adding the porcine pericardium modified with methacrylic anhydride to a zwitterion monomer solution having a final concentration as shown in a table below, and soaking overnight at 37°C; then adding an initiator for crosslinking reaction at 37°C for 24 hrs; obtaining the prepared porcine pericardium. The prepared porcine pericardium was thoroughly washed with deionized water and stored in a 25% isopropanol solution for later use.

Unmodified biological tissue: the unmodified biological tissue was prepared by the steps described above before the initiator is added for crosslinking, and the porcine pericardium is directly cross-linked by adding an initiator without being soaked in a monomer solution. The unmodified porcine pericardium was prepared, and the prepared porcine pericardium was thoroughly washed with deionized water and stored in a 25% isopropanol solution for later use.

Specific formulas are shown below:

| Sample name | Type and concentration of monomer | Initiator concentration | Functional molecule (MPC/SBMA) content (w/w) |
|---|---|---|---|
| MPC-20 | MPC: 20 mM | 50 mM ammonium persulfate and 50 mM sodium hydrogen sulfite | 1.6% |
| MPC-500 | MPC: 500 mM | 50 mM ammonium persulfate and 50 mM | 15.6% |
| | | sodium hydrogen sulfite | |
| SBMA-20 | SBMA: 20 mM | 50 mM ammonium persulfate and 50 mM sodium hydrogen sulfite | 5.2% |
| SBMA-500 | SBMA: 500 mM | 50 mM ammonium persulfate and 50 mM sodium hydrogen sulfite | 24.6% |
| Unmodified | 0 | 50 mM ammonium persulfate and 50 mM sodium hydrogen sulfite | 0%* |
| Note: SBMA: [2-(methylacryloyloxy)ethyl]dimethyl-(3-sulfopropyl)ammonium hydroxide;<br>MPC: 2-methylacryloyloxyethyl phosphorylcholine;<br>*: the unmodified is used as a reference line, set as 0%; and<br>the initiator concentration is the concentration initially added to the reaction system. | | | |

The above-mentioned functional molecule content is derived from analyzing by ICP-OES the content of sulfur or phosphorus in the biological material after freeze-dried since phosphorus element are contained in the functional molecule MPC and sulfur element are contained in SBMA, both of which are hardly found in the biological material. Thus, the degree of modification with the functional molecule on the biological tissue can be obtained, which facilitates the screening of experimental conditions.

The specific conversion formula is as follows:

$$\text{Functional molecule content} = \frac{C_{S(P)} \times 10 / M_{S(P)} \times M_{SBMA(MPC)}}{W} \times 100\%$$

wherein $C_{S(P)}$ (mg/L) is the concentration of sulfur or phosphorus in solution measured by ICP-OES, $M_{S(P)}$ is the relative atomic mass of sulfur or phosphorus, $M_{SBMA(MPC)}$ is the relative molecular mass of the functional molecule, and W (mg) is the dry weight of the biological material.

As can be seen from the above table, compared with the control group, the biological material prepared by the present invention shows that the biological tissue is well modified with the functional molecule, particularly, the SBMA-500 group has the highest degree of modification.

**Test Example 1: Stability of biological tissue**

The obtained biological material was cut into small pieces of 3-8 mg, aspirated with filter paper to remove water on the surface, and then placed in an aluminum crucible and sealed. The crucible with the sample was placed in the instrument and the thermal denaturation temperature was analyzed by differential scanning calorimetry, to characterize the stability of the biological tissue. The results are shown below:

| Sample name | Thermal denaturation temperature (°C) |
|---|---|
| SBMA-500 | 80.15 |
| Fresh tissue | 68.88 |

As can be seen from the above table, the biological material prepared by the present invention has a higher temperature resistance compared with fresh tissue, indicating that the biological tissue has excellent stability.

**Test Example 2: Anticoagulation performance of biological tissue**

The prepared biological material was washed, cut to have an appropriate size, and incubated with platelet-rich plasma for one hr. The amount of lactate dehydrogenase (LDH) after lysis of platelets adhering to the material was measured to express the amount of platelet adhered. The results are shown in a table below. The adhesion of platelets on the material was observed under SEM. The results are specifically shown in FIGs. 3-8.

| Sample name | Absorbency value (490 nm) |
|---|---|
| MPC-20 | 0.30 |
| MPC-500 | 0.29 |
| SBMA-20 | 0.24 |
| SBMA-500 | 0.110 |
| Unmodified | 0.32 |
| Crosslinked with glutaraldehyde | 0.35 |

As can be seen from the above table, a higher absorbency value at 490 nm indicates a higher lactate dehydrogenase content, that is, the amount of platelets adhered to the material is higher. Therefore, as can be seen from the above table, the anticoagulation effect of the biological material prepared by the present invention is apparently better than those of unmodified biological tissue and glutaraldehyde-crosslinked biological tissue (prepared by immersing porcine pericardium in 0.625 vol% of glutaraldehyde aqueous solution, crosslinking for 24 hrs at pH 7.4 and room temperature, and then dehydrating and drying). The anticoagulation effect of the biological tissue modified with SBMA is better than that of the biological tissue modified with MPC, and the anticoagulation effect of the biological tissue with a high grafting degree of SBMA is the best.

The qualitative observation results under SEM in FIGs. 3-8 are consistent with the quantitative measurement results of LDH.

**Test Example 3. Characterization of anti-calcification performance and immune reaction of biological tissue**

The prepared biological material was cut into pieces of 1 square centimeter, subcutaneously implanted in SD rats, and removed after 90 days. The calcium content in the samples was measured by ICP-OES. The results are shown below:

| Sample name | Calcium content (g/kg) |
|---|---|
| SBMA-500 | 12.8 |
| Unmodified | 38.4 |
| Crosslinked with glutaraldehyde | 117.3 |

As can be seen from the above table, the calcification of the biological tissue modified with SBMA-500 was significantly lower than those of unmodified biological tissue and glutaraldehyde-crosslinked biological tissue.

**Test Example 4: Recellularization performance of biological tissue**

The surface of the prepared biological material was seeded with endothelial cells, incubated for 3 days, and then stained with DAPI and FITC labeled phalloidin. The results are shown in FIGs. 9-11.

As can be seen from FIGs. 9-11, the growth of endothelial cells on the biological tissue modified with SBMA-500 was better than those on unmodified biological tissue and glutaraldehyde-crosslinked biological tissue.

**Test Example 5. Implantation of artificial blood vessels**

The prepared biological material was rolled into a tube with a diameter of about 2 mm and the junction was bonded with a medical adhesive, aspirated with filter paper to remove water on the surface, weighed and recorded. A rabbit carotid artery having a length of approximately 2 cm was replaced by the tube using vascular anastomosis. After

implantation for a period of time, it was retracted, observed, aspirated with filter paper to remove water on the material, and weighed again. The weight gain was calculated, which is the amount of thrombus produced. The result is shown below.

| Sample name | Initial weight (mg) | Final weight (mg) | Amount of thrombus produced (mg) |
|---|---|---|---|
| SBMA-500 | 80.9 | 83.6 | 2.7 |
| Unmodified | 78.6 | 262.4 | 183.8 |
| Crosslinked with glutaraldehyde | 82.5 | 295.3 | 212.8 |

From the above table, it can be seen that for glutaraldehyde-crosslinked biological tissue and unmodified biological tissue, thrombus of more than 2 times of the tissue weight is produced, but almost no thrombus is produced for SBMA-500.

## Claims

1. A method for preparing an anticoagulation and anticalcification biological material, comprising following steps: introducing, on a biological tissue, a polymerizable reactive group, and undergoing free radical copolymerization with a zwitterion, wherein the zwitterion has a specific structure as below:

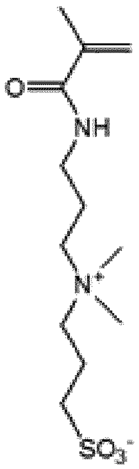
,
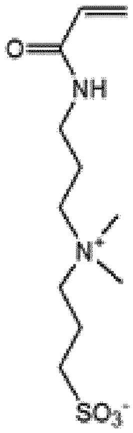
,
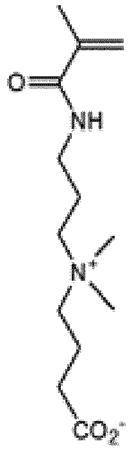
,
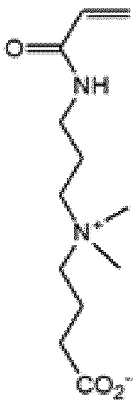
,
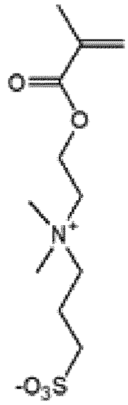
,
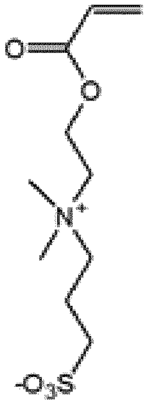
,
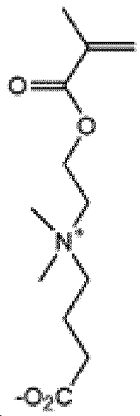
,
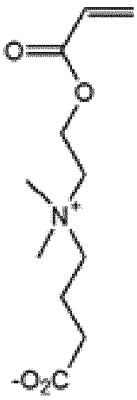
or
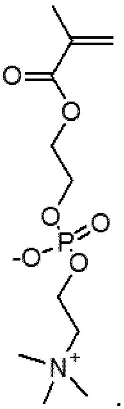
.

2. The method for preparing an anticoagulation and anticalcification biological material according to claim 1, wherein the biological tissue introduced with the reactive group is added to a zwitterion solution having a final concentration of 20 to 500 mM, and an initiator is added to initiate the polymerization, to obtain the anticoagulation and anticalcification biological material.

3. The method for preparing an anticoagulation and anticalcification biological material according to claim 1, wherein the biological tissue introduced with the reactive group is added to a zwitterion solution and soaked at 35 to 40°C, and an initiator is added to initiate the polymerization, to obtain the anticoagulation and anticalcification biological material.

4. The method for preparing an anticoagulation and anticalcification biological material according to claim 1, wherein the biological tissue is soaked in deionized water, and then the reactive group is added, to provide a concentration of the reactive group of 3-10% by volume.

5. The method for preparing an anticoagulation and anticalcification biological material according to claim 4, wherein the biological tissue is reacted with the reactive group at room temperature for 12 to 36 hrs, and then the biological tissue is washed.

6. The method for preparing an anticoagulation and anticalcification biological material according to claim 4, wherein the biological tissue is soaked in deionized water, and then the reactive group is added to provide a concentration of the reactive group of 4% by volume. and then reacted at room temperature for 24 hrs.

7. The method for preparing an anticoagulation and anticalcification biological material according to any one of claims 1 to 6, wherein the zwitterion solution is added to the washed biological tissue, to provide a final concentration of the zwitterion of 500 mM, and the biological tissue is soaked at 37°C.

8. The method for preparing an anticoagulation and anticalcification biological material according to any one of claims 1 to 6, wherein the biological tissue is pericardium, aortic root, aortic valve, pulmonary artery root, pulmonary artery valve, tendon, ligament, skin, dura mater, peritoneum, blood vessels, pleura, septum, mitral or tricuspid valve.

9. The method for preparing an anticoagulation and anticalcification biological material according to any one of claims 1 to 6, wherein the reactive group is methacrylic anhydride, acrylamide, methacrylamide, acrylate, methacrylate or allyl.

10. The method for preparing an anticoagulation and anticalcification biological material according to any one of claims 1 to 6, wherein the zwitterion accounts for 1 to 30% by weight based on a total weight of the anticoagulation and anticalcification biological material.

11. The method for preparing an anticoagulation and anticalcification biological material according to claim 2 or 3, wherein when the initiator initiates the polymerization, a reaction temperature is not higher than 50°C.

12. The method for preparing an anticoagulation and anticalcification biological material according to claim 2 or 3, wherein the initiator is a thermal initiator or a photoinitiator.

13. An anticoagulation and anticalcification biological material prepared by the preparation method according to any one of claims 1 to 12.

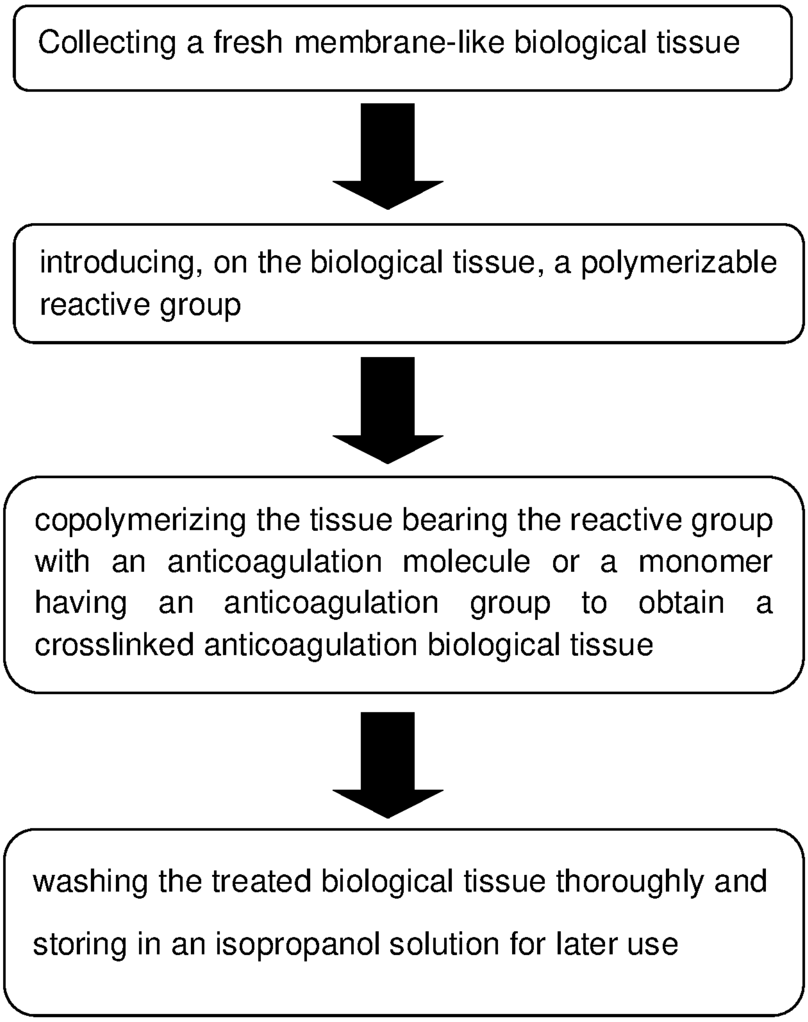
Collecting a fresh membrane-like biological tissue
introducing, on the biological tissue, a polymerizable reactive group
copolymerizing the tissue bearing the reactive group with an anticoagulation molecule or a monomer having an anticoagulation group to obtain a crosslinked anticoagulation biological tissue
washing the treated biological tissue thoroughly and storing in an isopropanol solution for later use

FIG. 1

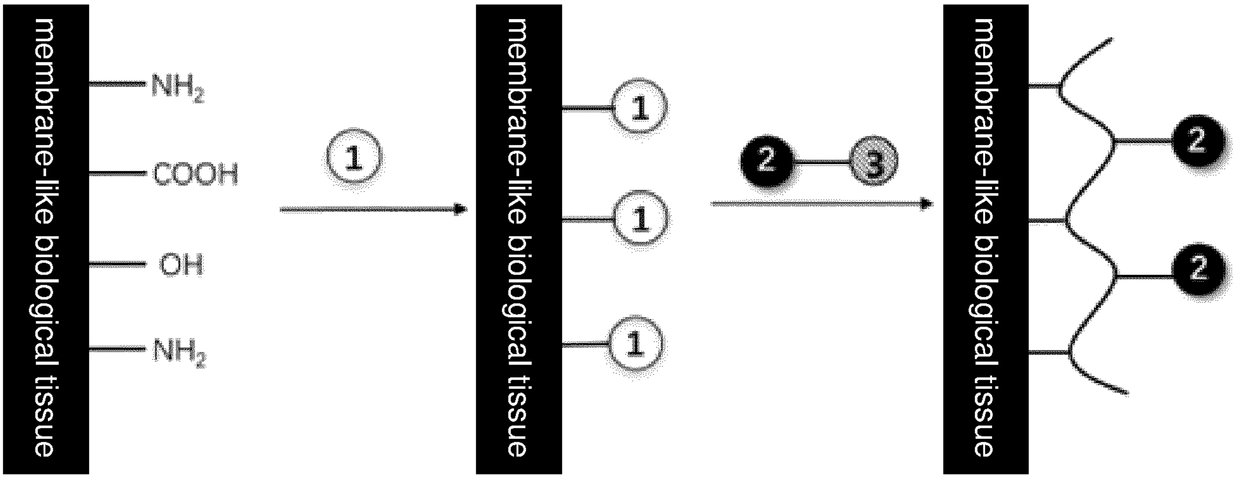
membrane-like biological tissue
NH2
COOH
OH
NH2
1
membrane-like biological tissue
1
1
1
2
3
membrane-like biological tissue
2
2

FIG. 2

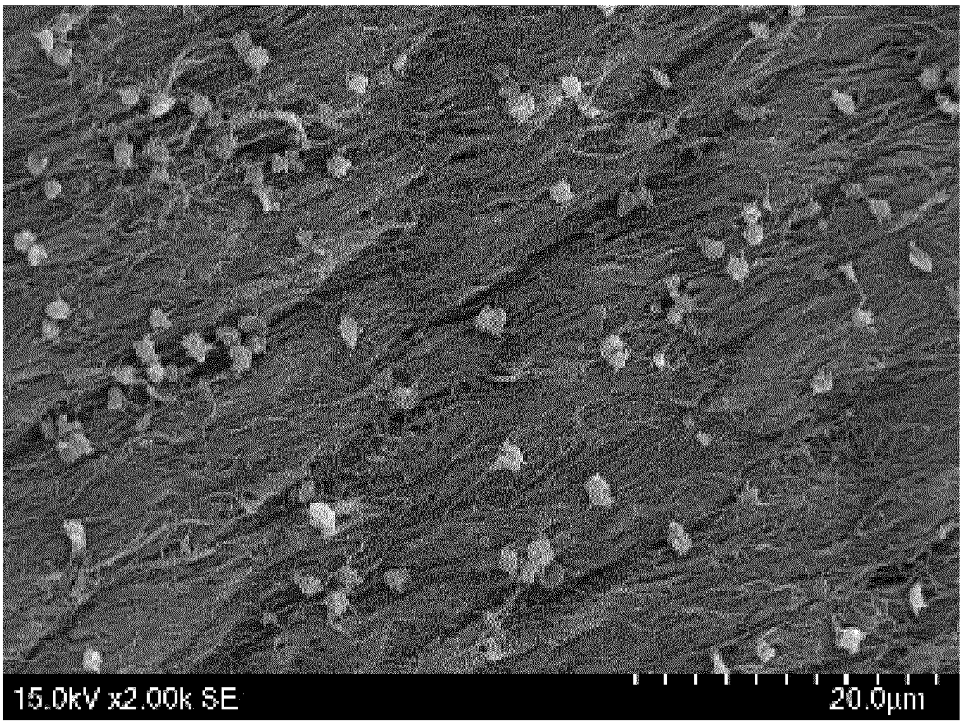
15.0kV x2.00k SE
20.0μm

FIG. 3

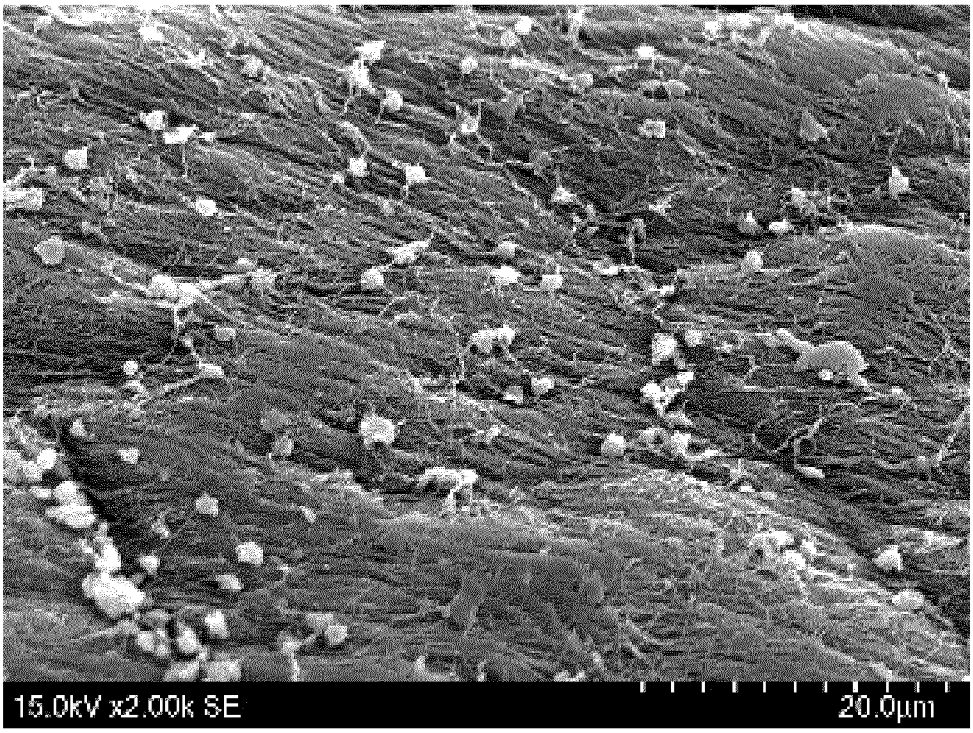


FIG. 4

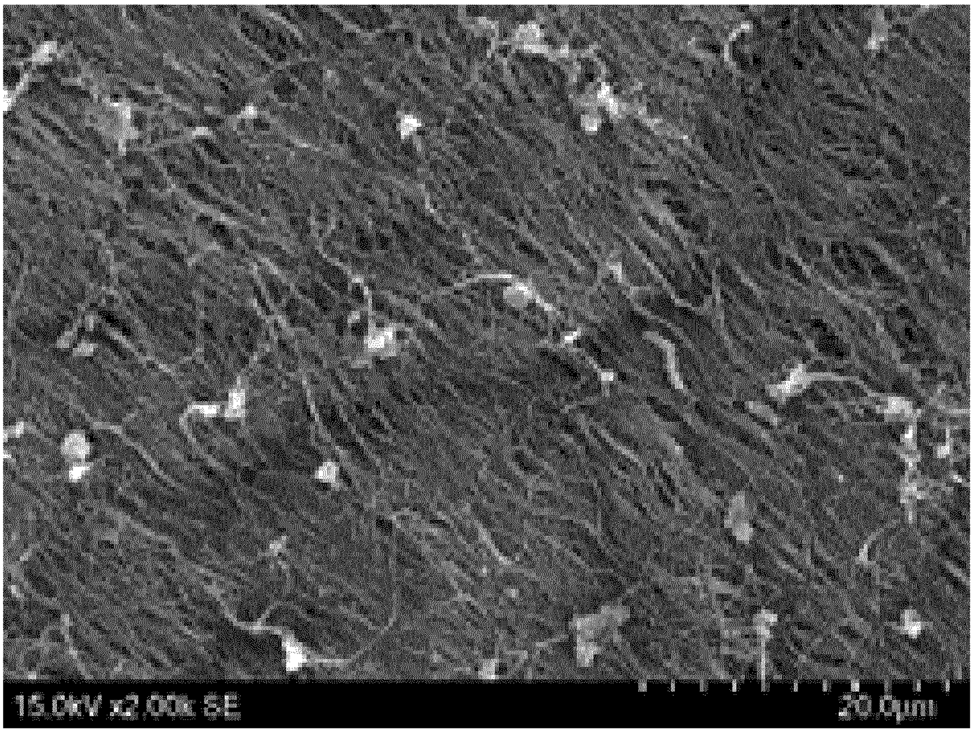


FIG. 5

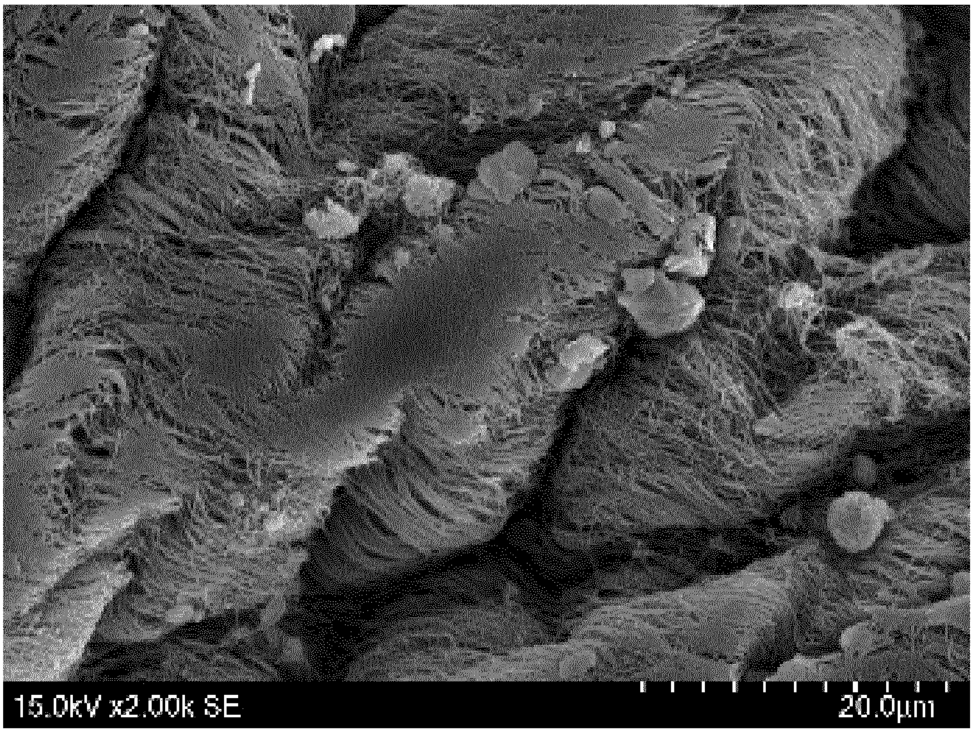


FIG. 6

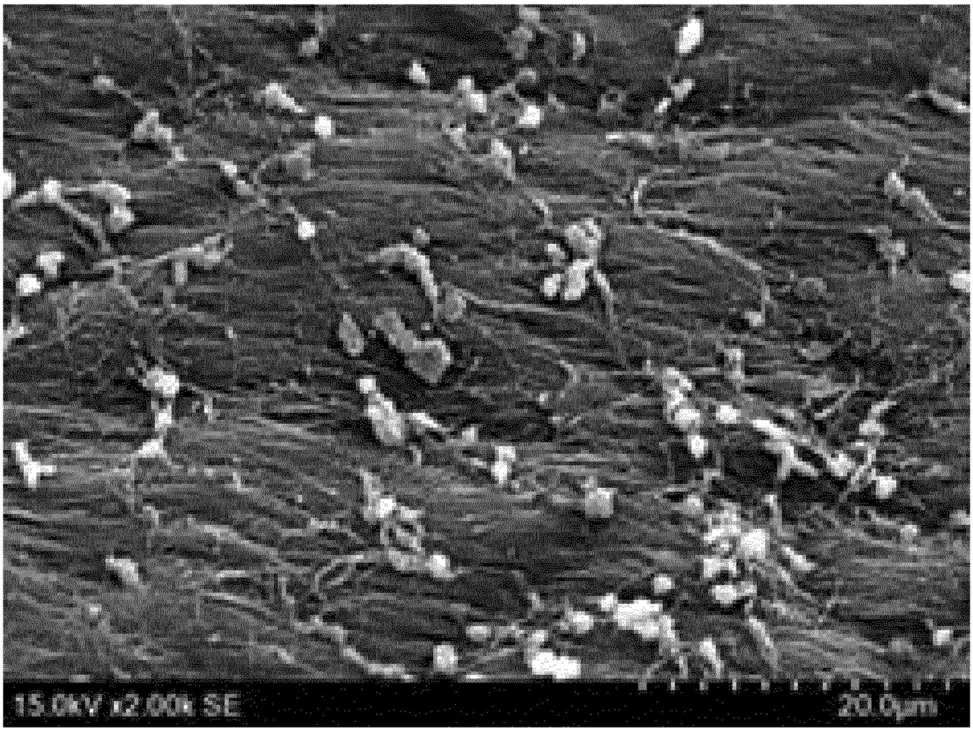


FIG. 7

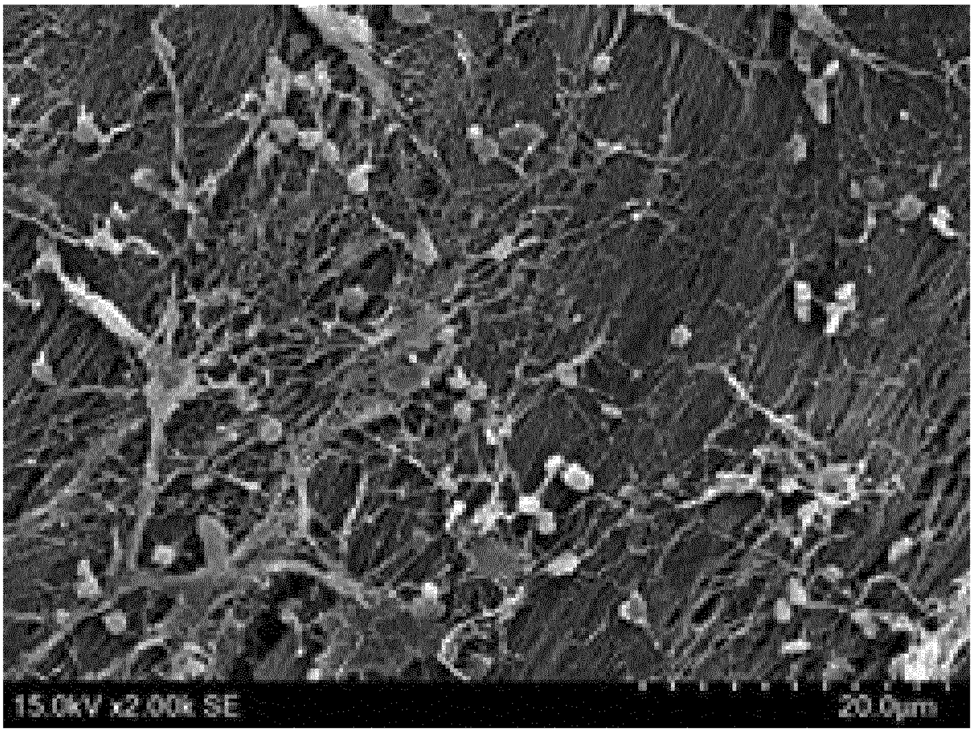


FIG. 8

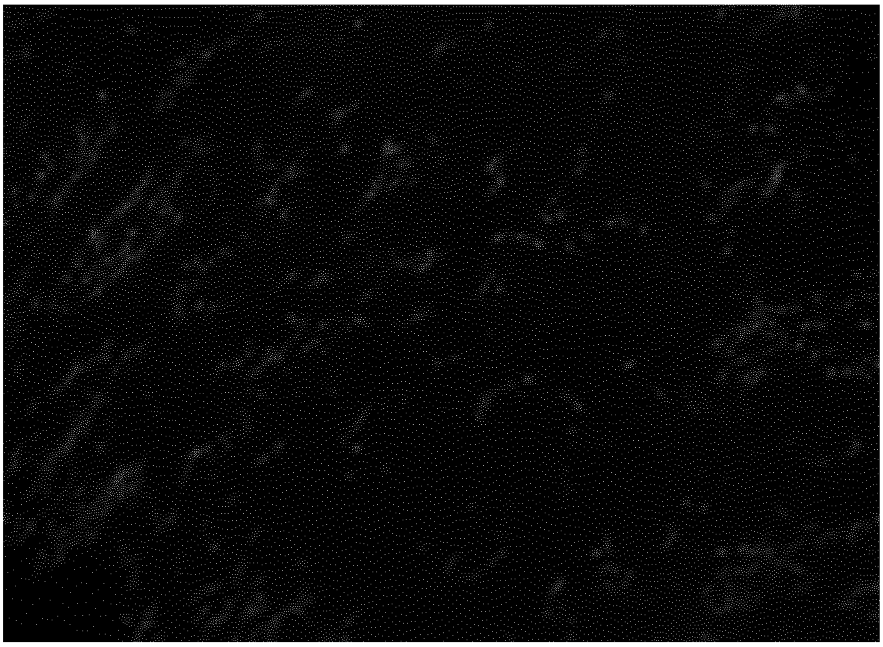

FIG. 9

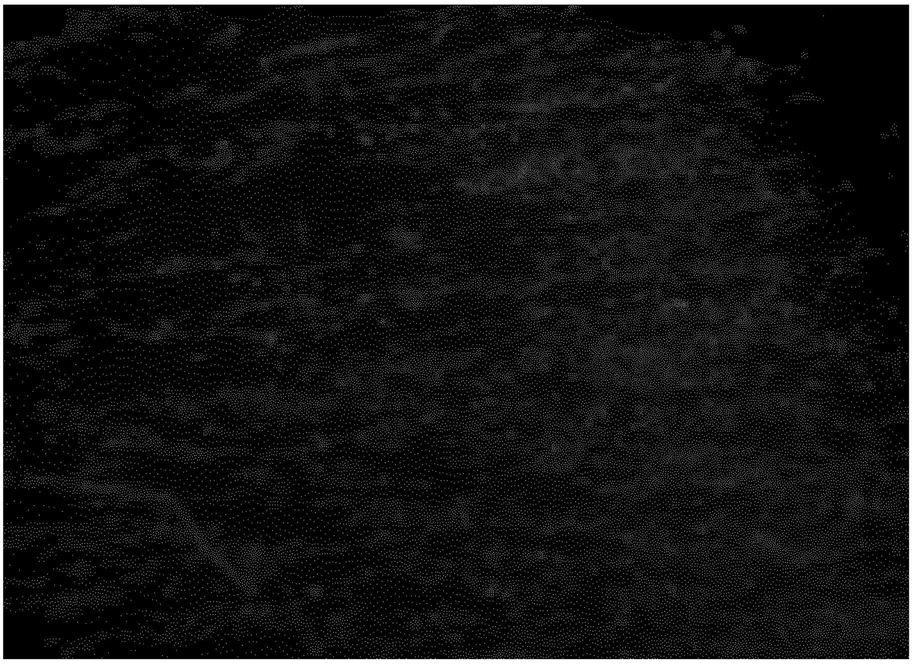

FIG. 10

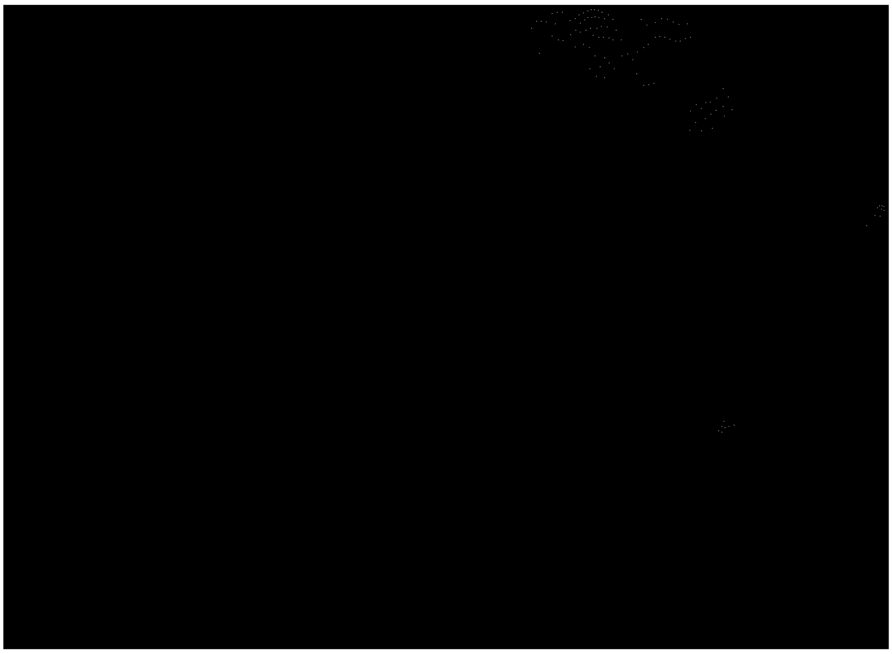

FIG. 11

**INTERNATIONAL SEARCH REPORT**

International application No.
**PCT/CN2021/089448**

**A. CLASSIFICATION OF SUBJECT MATTER**

A61L 27/36(2006.01)i；A61L 27/24(2006.01)i；A61L 33/00(2006.01)i；C08F 289/00(2006.01)i；C08F 220/60(2006.01)i；C08F 220/36(2006.01)i；C08F 230/02(2006.01)i

According to International Patent Classification (IPC) or to both national classification and IPC

**B. FIELDS SEARCHED**

Minimum documentation searched (classification system followed by classification symbols)

A61L，C08F

Documentation searched other than minimum documentation to the extent that such documents are included in the fields searched

Electronic data base consulted during the international search (name of data base and, where practicable, search terms used)

DWPI, SIPOABS, CNABS, CNTXT, CNKI: 心包膜, 瓣膜, 活性基团, 甲基丙烯酸酐, 丙烯酰胺, 丙烯酸酯, 丙烯基, 抗凝血, 抗钙化, 两性离子, SBMA , CBMA, MPC, pericardium, valve, active group, methacrylic anhydride, acrylamide, acrylate, allyl, anticoagulant+, anti?calcificat+, zwitter-ion

**C. DOCUMENTS CONSIDERED TO BE RELEVANT**

| Category* | Citation of document, with indication, where appropriate, of the relevant passages | Relevant to claim No. |
|---|---|---|
| PX | CN 111481743 A (SICHUAN UNIVERSITY) 04 August 2020 (2020-08-04)<br>claims 1-10, description paragraphs [0021]-[0025] | 1-13 |
| Y | Gaoyang Guo et al. "Radical polymerization-crosslinking method for improving extracellular matrix stability in bioprosthetic heart valves with reduced potential for calcification and inflammatory response"<br>*Acta Biomaterialia,* Vol. 82, 14 October 2018 (2018-10-14),<br>abstract, page 45 left-hand column “Mterials and methods” in part | 1-13 |
| Y | Wei-Hsuan Kuo et al. "Surface Modification with Poly(Sulfobetaine Methacrylate-co-acrylic Acid) To Reduce Fibrinogen Adsorption, Platelet Adhesion, and Plasma Coagulation"<br>*Biomacromolecules,* Vol. 12, No. 12, 13 November 2011 (2011-11-13),<br>page 4349, section 2.3, page 4350 section 2.5, page 4355 section 5 | 1-13 |
| Y | CN 109820625 A (SICHUAN UNIVERSITY) 31 May 2019 (2019-05-31)<br>description, paragraphs [0006]-[0022] | 1-13 |
| A | CN 101128225 A (CELXCEL PTY LTD.) 20 February 2008 (2008-02-20)<br>entire document | 1-13 |

☑ Further documents are listed in the continuation of Box C. ☑ See patent family annex.

* Special categories of cited documents:
“A” document defining the general state of the art which is not considered to be of particular relevance
“E” earlier application or patent but published on or after the international filing date
“L” document which may throw doubts on priority claim(s) or which is cited to establish the publication date of another citation or other special reason (as specified)
“O” document referring to an oral disclosure, use, exhibition or other means
“P” document published prior to the international filing date but later than the priority date claimed
“T” later document published after the international filing date or priority date and not in conflict with the application but cited to understand the principle or theory underlying the invention
“X” document of particular relevance; the claimed invention cannot be considered novel or cannot be considered to involve an inventive step when the document is taken alone
“Y” document of particular relevance; the claimed invention cannot be considered to involve an inventive step when the document is combined with one or more other such documents, such combination being obvious to a person skilled in the art
“&” document member of the same patent family

| Date of the actual completion of the international search | Date of mailing of the international search report |
|---|---|
| **06 July 2021** | **21 July 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

INTERNATIONAL SEARCH REPORT

International application No. PCT/CN2021/089448

C. DOCUMENTS CONSIDERED TO BE RELEVANT

| Category* | Citation of document, with indication, where appropriate, of the relevant passages | Relevant to claim No. |
|---|---|---|
| A | CN 107617126 A (TIANJIN UNIVERSITY) 23 January 2018 (2018-01-23)<br>entire document | 1-13 |

Form PCT/ISA/210 (second sheet) (January 2015)

**INTERNATIONAL SEARCH REPORT**
**Information on patent family members**

International application No.
**PCT/CN2021/089448**

| Patent document cited in search report | | | Publication date (day/month/year) | Patent family member(s) | | | Publication date (day/month/year) |
|---|---|---|---|---|---|---|---|
| CN | 111481743 | A | 04 August 2020 | CN | 111481743 | B | 30 March 2021 |
| CN | 109820625 | A | 31 May 2019 | None | | | |
| CN | 101128225 | A | 20 February 2008 | BR | PI0519285 | B1 | 15 May 2018 |
| | | | | DK | 1835948 | T3 | 30 May 2016 |
| | | | | EP | 1835948 | B1 | 24 February 2016 |
| | | | | US | 2006193885 | A1 | 31 August 2006 |
| | | | | IL | 184130 | D0 | 31 October 2007 |
| | | | | WO | 2006066327 | A1 | 29 June 2006 |
| | | | | EA | 200701377 | A1 | 28 February 2008 |
| | | | | KR | 20070106696 | A | 05 November 2007 |
| | | | | PL | 1835948 | T3 | 30 December 2016 |
| | | | | JP | 2008525057 | A | 17 July 2008 |
| | | | | JP | 5208513 | B2 | 12 June 2013 |
| | | | | EP | 1835948 | A1 | 26 September 2007 |
| | | | | AU | 2005318938 | A1 | 29 June 2006 |
| | | | | ES | 2569494 | T3 | 11 May 2016 |
| | | | | ZA | 200706089 | A | 29 October 2008 |
| | | | | BR | PI0519285 | A2 | 04 August 2009 |
| | | | | CA | 2591882 | C | 15 October 2013 |
| | | | | NO | 20073846 | L | 17 September 2007 |
| | | | | ZA | 200706089 | B | 29 October 2008 |
| | | | | EP | 1835948 | B8 | 06 April 2016 |
| | | | | NO | 20073846 | A | 17 September 2007 |
| | | | | EP | 1835948 | A4 | 30 November 2011 |
| | | | | US | 9205172 | B2 | 08 December 2015 |
| | | | | MX | 2007007723 | A | 07 December 2007 |
| | | | | SG | 158172 | A1 | 29 January 2010 |
| | | | | AU | 2005318938 | B2 | 21 April 2011 |
| | | | | CN | 101128225 | B | 15 June 2011 |
| | | | | NZ | 556610 | A | 26 November 2010 |
| | | | | CA | 2591882 | A1 | 29 June 2006 |
| CN | 107617126 | A | 23 January 2018 | None | | | |

Form PCT/ISA/210 (patent family annex) (January 2015)